# EUROPEAN PATENT SPECIFICATION

(11) **EP 2 591 758 B1**
(45) Date of publication and mention of the grant of the patent: **17.11.2021**
(21) Application number: 11803583.1
(22) Date of filing: 05.07.2011
(51) Int. Cl.: A61F 13/49, A61F 13/53

(54) **DISPOSABLE DIAPER**
WEGWERFWINDEL
COUCHE JETABLE

(30) Priority: 03.08.2010 JP 2010174632; 07.07.2010 JP 2010154784
(43) Date of publication of application: 15.05.2013
(73) Proprietor: Kao Corporation, Tokyo 103-8210 (JP)
(72) Inventor: YAMAMOTO, Ryoichi, Haga-gun Tochigi 321-3497 (JP); SONO, Tokihito, Haga-gun Tochigi 321-3497 (JP); OKUDA, Yasuyuki, Haga-gun Tochigi 321-3497 (JP)
(74) Representative: Vossius & Partner Patentanwälte Rechtsanwälte mbB
(86) International application number: PCT/JP2011/065382
(87) International publication number: WO 2012/005249

(56) References cited:
- EP-A2- 0 241 041
- WO-A1-01/24755
- WO-A1-2008/069279
- JP-A- 2005 312 557
- JP-A- 2006 081 653
- JP-A- 2007 029 507
- JP-A- 2007 117 391
- JP-A- 2007 144 105
- JP-A- 2008 183 160
- JP-A- 2008 284 190
- None

## Description

### Technical Field

The present invention relates to a disposable diaper.

### Background Art

An absorbent member having been widely used in disposable diapers includes an absorbent core formed of a fiber aggregate made of a fibrous material, such as defibrated pulp, with or without a functional material, such as an absorbent polymer, incorporated therein, and a core wrap sheet, such as tissue or water-pervious nonwoven fabric, wrapping the absorbent core.

In order to provide an absorbent member with improved deformability so as to give improved fit and protection against leakage, proposals have been made in which an absorbent core is provided with a slit or a region where the core-forming material is absent.

For example, EP 2 092 922 A1 discloses an absorbent article having a chassis and an absorbent member, the chassis having a front torso surrounding region and a rear torso surrounding region, and the absorbent member straddling the front and the rear torso surrounding region, wherein an absorbent core of the absorbent member has a low-rigidity central portion and a pair of low-rigidity side portions. JP-U 02-010824 A and JP 2008-284190 A disclose an absorbent core for a disposable diaper having three regions devoid of the core-forming material or slits in juxtaposed relation. JP 2008-284190 A discloses the features recited in the preamble of claim 1.

EP 0 241 041 A and WO 01/24755 A1 show other absorbent articles

### Summary of Invention

In EP 2 092 922 A1, the absorbent core in the crotch region is described as capable of bending to have a "W"-shaped transverse cross-section. However, because the low-rigidity central portion is so long as to straddle the front torso surrounding region and the rear torso surrounding region, a space enough to contain feces is not easily provided between the wearer's skin and the skin-facing surface of the absorbent article, which can result in inconveniences such as discomfort due to the adhesion of feces to the skin and leakage from the back side of the absorbent article.

In the disposable diaper of JP-U 02-010824 A having three juxtaposed regions devoid of a core-forming material, the three regions lie with their front ends as well as their rear ends even with each other. Therefore, a concave space for holding feces is not easily formed near the wearer's anus, and using the regions devoid of a core-forming material to guide a bodily fluid toward the front portion may cause a fluid to leak from the lateral side of the front portion or vicinities thereof.

The disposable diaper of JP 2008-284190 A has an elongated slit located in the laterally middle part of the absorbent core and two slits each located in both side of the elongated slit, two slits extending beyond the end of the elongated slit in the front portion side of the absorbent core. Therefore, using those slits to guide a bodily fluid toward the front portion may cause a fluid to leak from the lateral side of the front portion or vicinities thereof.

The invention relates to a disposable diaper that provides excellent protection against urine and feces leakage and reduces discomfort of having feces adhering to the skin.

The invention provides a disposable diaper as defined in independent claim 1 with its dependent claims.

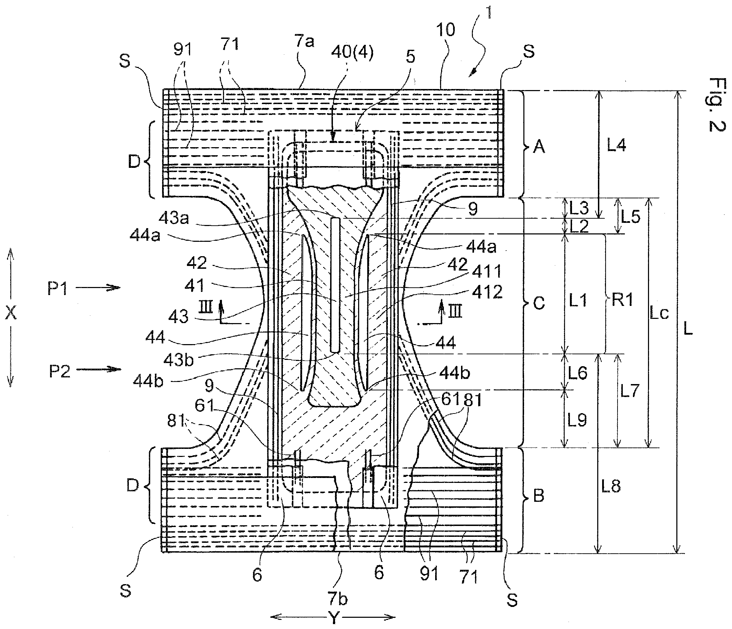

The invention provides disposable diapers described below.
[1] A disposable diaper according to the invention is specified in claim 1.
[2] The disposable diaper described in [1], wherein the high basis weight region has a width increasing from an area where the central bending-inducing region and the side bending-inducing regions are in juxtaposition toward the longitudinal end of the front portion.
[3] The disposable diaper described in [1] or [2], wherein the front end of the central bending-inducing region is short of the front portion.
[4] The disposable diaper described in any one of [1] to [3], wherein the rear end of each side bending-inducing region is beyond the rear end of the central bending-inducing region by a distance L6 of 20 to 120 cm.
[5] The disposable diaper described in any one of [1] to [4], wherein the rear end of the central bending-inducing region is at or short of the position corresponding to the wearer's exit point of feces, and the rear end of each bending-inducing region is beyond the position corresponding to the wearer's exit point of feces.
[6] The disposable diaper described in any one of [1] to [5], wherein the absorbent core comprises an upper absorbent layer and a lower absorbent layer larger than the upper absorbent layer in both the longitudinal and the transverse direction, the high basis weight region is a region where the upper and the lower absorbent layer overlap, the low basis weight regions are each a region where the upper and the lower absorbent layer do not overlap, and the central bending-inducing region is a rectangular through-hole that is longer in the longitudinal direction of the absorbent core and pierces through the upper and the lower absorbent layer.
[7] The disposable diaper described in [6], wherein the through-hole is the result of stacking the upper and the lower absorbent layers each having a through-hole with their through-holes aligned with each other.
[8] The disposable diaper described in any one of [1] to [7], wherein the absorbent core has a multi-layered structure including a lower absorbent layer having a fiber-free region (a through-hole rectangular longer in the longitudinal direction of the absorbent core in a plan view) and an upper absorbent layer having a slit narrower than the fiber-free region, and the central bending-inducing region is the result of stacking the upper and the lower absorbent layer with the fiber-free region and the slit aligned with each other.
[9] The disposable diaper described in any one of [1] to [7], wherein the absorbent core has a multi-layered structure including an upper absorbent layer having a fiber-free region (a through-hole rectangular longer in the longitudinal direction of the absorbent core in a plan view) and a lower absorbent layer having a slit narrower than the fiber-free region, and the central bending-inducing region is the result of stacking the upper and the lower absorbent layer with the fiber-free region and the slit aligned with each other.
[10] The disposable diaper described in any one of [1] to [7], wherein the central bending-inducing region is a groove formed as a result of stacking an upper absorbent layer having a through-hole longer in the longitudinal direction of the absorbent core in a plan view and a lower absorbent layer having no through-hole.
[11] The disposable diaper described in any one of [1] to [10], wherein the absorbent core has a single-layer structure, and the high basis weight region is a thickened part of the absorbent core.
[12] The disposable diaper described in any one of [1] to [11], wherein the absorbent core has a single-layer structure, and the central bending-inducing region is a groove formed by embossing the absorbent core.
[13] The disposable diaper described in any one of [1] to [12], wherein the absorbent core has, in its crotch portion, a juxtaposition region where the central bending-inducing region and the side bending-inducing regions are in juxtaposition to the longitudinal direction of the absorbent core.
[14] The disposable diaper described in [13], wherein the length L1 (see Fig. 2) of the juxtaposition region in the longitudinal direction of the absorbent core is 20% to 80% of the length Lc (see Fig. 2) of the crotch portion in the longitudinal direction of the diaper.
[15] The disposable diaper described in any one of [1] to [14], wherein the front end of the central bending-inducing region is beyond the front end of each side bending-inducing region by a distance L2 (see Fig. 2) of 5 to 40 cm.
[16] The disposable diaper described in any one of [1] to [15], wherein the front end of the central bending-inducing region is short of the front portion.
[17] The disposable diaper described in any one of [1] to [16], wherein the front end of the central bending-inducing region is short of the front portion by a distance L3 (see Fig. 2) of 5 cm or more.
[18] The disposable diaper described in any one of [1] to [17], wherein the front end of the central bending-inducing region is short of the front portion by a distance (see Fig. 2) of 10 to 20 cm.
[19] The disposable diaper described in any one of [1] to [18], wherein the side bending-inducing regions each extend beyond the rear end of the central bending-inducing region by a distance L6 (see Fig. 2) of 20 to 120 cm.
[20] The disposable diaper described in any one of [1] to [19], wherein the rear end of each side bending-inducing region is short of the rear portion.
[21] The disposable diaper described in any one of [1] to [20], wherein the central bending-inducing region in the form of a slit, a groove, or a fiber-free region is partly discontinuous.
[22] The disposable diaper described in any one of [1] to [21], wherein the fiber-free region as each side bending-inducing region is partly discontinuous.
[23] The disposable diaper described in any one of [1] to [22], wherein the absorbent core has, in the crotch portion, a central absorbent portion extending in its longitudinal direction and a pair of side absorbent portions located on both lateral sides of the central absorbent portion, and the central absorbent portion contains the absorbent polymer in a higher basis weight than each side absorbent portion.
[24] The disposable diaper described in any one of [1] or [23], wherein the central absorbent portion has a multi-layered structure comprising an upper absorbent layer and a lower absorbent layer, and the upper absorbent layer contains the absorbent polymer in a higher basis weight than the lower absorbent layer.
[25] The disposable diaper described in any one of [1] to [24], being of pull-on type, comprising an absorbent assembly having the absorbent member and an outer cover located on the non-skin-facing side of the absorbent assembly and having the absorbent assembly fixed thereto, the outer cover having its opposite lateral side edges in the front portion joined to those in the rear portion to form a pair of side seals, a waist opening, and a pair of leg openings.
[26] The disposable pull-on diaper described in [25], being of separate outer cover type wherein the outer cover is separated into a rear side panel adapted to be worn about the rear side of a wearer and a front side panel adapted to be worn about the front side of a wearer, the rear side panel and the front side panel being joined to each other along the pair of side seals to form a cylindrical shape, and the absorbent assembly being fixed to bridge the front side panel and the rear side panel.
[27] The disposable diaper described in any one of [1] to [25], being of flat type having a fastening tape in the rear portion and a landing tape receiving the fastening tape in the outer side of the front portion.

### Brief Description of Drawings

[Fig. 1] Fig. 1 is a perspective of a disposable pull-on diaper not part of the present invention.
[Fig. 2] Fig. 2 is a plan of the disposable pull-on diaper of Fig. 1 in its flat-out, uncontracted state, seen from the skin facing side, with a part cut away. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 3] Fig. 3 is a cross-section taken along line III-III in Fig. 2.
[Fig. 4] Fig. 4(a) and Fig. 4(b) are each a schematic transverse cross-section of the absorbent core of the disposable diaper while worn, of which Fig. 4(a) is a cross-section taken at the position indicated by arrow P1 in Fig. 2, and Fig. 4(b) is a cross-section taken at the position indicated by arrow P2 in Fig. 2.
[Fig. 5] Fig. 5 is a perspective of a disposable pull-on diaper according to a first embodiment of the invention.
[Fig. 6] Fig. 6 is a plan of the disposable pull-on diaper of Fig. 5 in its flat-out, uncontracted state, seen from the skin facing side, with a part cut away.
[Fig. 7] Fig. 7 is a cross-section taken along line IV-IV of Fig. 6.
[Fig. 8] Fig. 8(a) and Fig. 8(b) are each a schematic transverse cross-section of the absorbent core of the disposable diaper of the first embodiment while worn, of which
Fig. 8(a) is a cross-section taken at the position indicated by arrow P1 in Fig. 6, and Fig. 8(b) is a cross-section taken at the position indicated by arrow P2 in Fig. 6.
[Fig. 9] Fig. 9 is a perspective view illustrating a preferred embodiment of the production of a disposable diaper according to the invention.
[Fig. 10] Fig. 10 is a plan view illustrating a preferred embodiment the production of a disposable diaper according to the invention.
[Fig. 11] Fig. 11 is a transverse cross-section of an absorbent member used in a second embodiment of the invention.

### Description of Embodiments

The invention will be described based on its preferred embodiments with reference to the accompanying drawings.

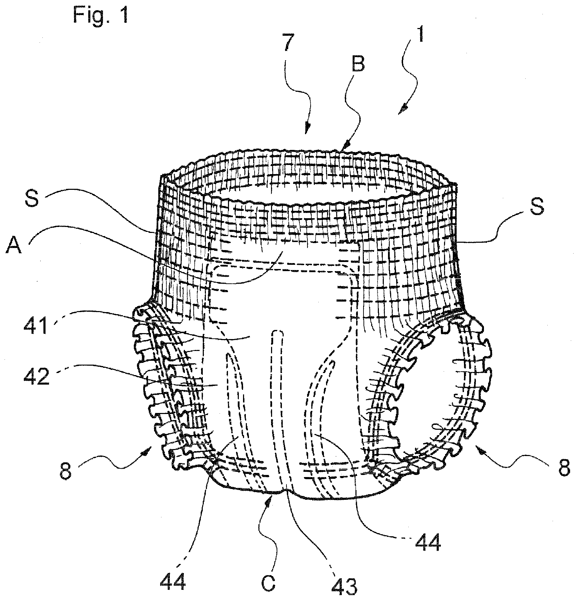

A disposable diaper 1 (hereinafter also simply referred to as a diaper 1) according to an example is of pull-on type. As shown in Figs. 1 through 3, the disper 1 includes an absorbent assembly 5 and an outer cover 10 located on the non-skin-facing side of the absorbent assembly 5 and having the absorbent assembly 5 fixed thereto. The absorbent assembly 5 includes a liquid permeable topsheet 2, a liquid impermeable or water repellent backsheet 3, and an oblong absorbent member 4 interposed between the sheets 2 and 3. The absorbent member 4 includes an absorbent core 40 that is oblong in the longitudinal direction of the absorbent member 4.

As shown in Fig. 1 and 2, the diaper 1 has a front portion A, a crotch portion B, and a rear portion C adapted to be worn about the front side, the crotch, and the rear side, respectively, of a wearer. The outer cover 10 has its opposite side edges in the front portion A joined to those in the rear portion B to form a pair of side seals S, S, a waist opening 7, and a pair of leg openings 8, 8.

The front portion A and the rear portion B of the disposable pull-on diaper are each defined to be a portion having the side seal S along both lateral side edges (opposite in the direction Y in Fig. 2). The longitudinal direction of the diaper is the direction extending from the front portion A through the crotch portion C to the rear portion B or the opposite direction, which is the direction X in Fig. 2. In the diaper 1 of the present example, all of the longitudinal direction of the diaper 1, the longitudinal direction of the absorbent member 4, the longitudinal direction of the absorbent core 40, and the longitudinal direction of a hereinafter described high basis weight region 41 are the direction X in Fig. 2.

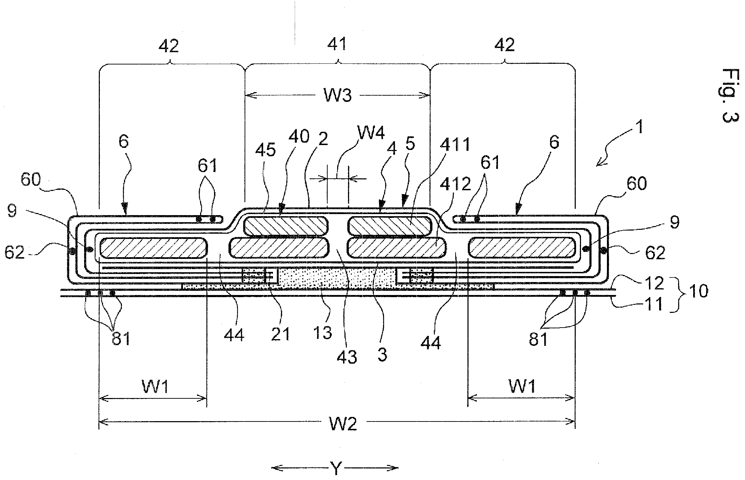

As shown in Fig. 2 and 3, the absorbent core 40 of the diaper 1 has a high basis weight region 41 in the laterally middle part of the crotch portion C and a low basis weight region 42 with a lower basis weight than that of the high basis weight region 41 on both sides of the high basis weight region 41. The high basis weight region 41 extends in the longitudinal direction of the diaper 1. In detail, as shown in Fig. 2, the absorbent core 40 includes a generally T-shaped upper absorbent layer 411 having a larger width in its front end portion than in its rear end portion in a plan view and a rectangular lower absorbent layer 412 larger than the upper absorbent layer 411 in both the longitudinal and the transverse direction. The overlap of the upper absorbent layer 411 and the lower absorbent layer 412 provides the high basis weight region 41, while the part of the lower absorbent layer 412 not having the upper absorbent layer 411 stacked thereon provides the low basis weight region 42.

The upper absorbent layer 411 extends through the crotch portion C and terminates in the front portion A. Each of the upper absorbent layer 411 and the high basis weight region 41 has their width gradually increased from about the longitudinally middle of the crotch portion C toward the front end of the front portion A. The lower absorbent layer 412 extends through the crotch portion C and terminates in the front portion A and the rear portion B.

As shown in Fig. 2, the absorbent core 40 of the diaper 1 has a fiber-free region extending in its longitudinal direction in the laterally middle part of the high basis weight region 41 to provide a central bending-inducing region 43. The absorbent core 40 also has a fiber-free region as a side bending-inducing region 44 in each of the low basis weight regions 42. Each side bending-inducing region 44 is located between the respective longitudinal side edge of the high basis weight region 41 and the longitudinal side edge of the absorbent core 40 and closer to the high basis weight region 41 than to the side edge of the absorbent core 40. Each side bending-inducing region 44 extends along the respective side edge of the high basis weight region 41.

The upper absorbent layer 411 and the lower absorbent layer 412 making up the absorbent core 40 of the present example are each formed of a fiber aggregate made of a fibrous material, such as defibrated pulp, with or without a functional material, such as a particulate absorbent polymer, incorporated therein. The absorbent member 4 is formed by totally covering the upper absorbent layer 411 and the lower absorbent layer 412 thus configured with a core wrap sheet 45. The core wrap sheet may be of various known materials, preferably thin paper (e.g., tissue) or water impervious nonwoven fabric. The absorbent member 4 may be formed by stacking the upper absorbent layer 411 and the lower absorbent layer 412 each of which has been separately wrapped in a core wrap sheet. In that case, the core wrap sheet wrapping the upper absorbent layer 411 and the core wrap sheet wrapping the lower absorbent layer 412 may or may not be bonded to each other.

As used herein, the term "fiber-free region" means a region having no material forming the absorbent core (core-forming material). Nevertheless, a region having a smaller amount (e.g., about 20 g/m²) of a core-forming material than in the low basis weight region 42 is included under the term "fiber-free region" because the same effects as by the above-defined "fiber-free region" are exerted. It should be noted that the amount of the core-forming material present in the fiber-free region is preferably 50 g/m² or less.

As shown in Figs. 2 and 3, the fiber-free region as the central bending-inducing region 43 is a rectangular through-hole longer in the longitudinal direction of the absorbent core piercing both the upper and the lower absorbent layer 411 and 412.

The through-hole piercing the upper and the lower absorbent layer 411 and 412 may be formed by making an upper and a lower absorbent layer each having a through-hole and stacking them with their through-holes aligned with each other; by making an upper and a lower absorbent layer each having no through-hole, cutting a through-hole in each of them, and stacking them with their through-holes aligned with each other; or by making an upper and a lower absorbent layer each having no through-hole, stacking them, and cutting a through-hole piercing the stacked layers.

In the present example, the through-hole of the upper absorbent layer 411 and that of the lower absorbent layer 412 coincide with each other in shape and position in a plan view. Instead of this, the two through-holes may be different in length and/or position in the longitudinal direction of the absorbent core. In the latter case, the positions of the longitudinal ends of the through-hole on the skin facing side define the positions of the longitudinal ends of the central bending-inducing region 43.

The central bending-inducing region 43 and the side bending-inducing regions 44, 44 are to increase the bending properties of the absorbent core 40 in the transverse direction.

The central bending-inducing region 43 may be a slit or a groove extending in the longitudinal direction of the absorbent core 40 in place of the fiber-free region. In the case where the absorbent core 40 has a multi-layered structure composed of the upper and the lower absorbent layer 411 and 412, the central bending-inducing region 43 may be formed by providing the upper absorbent layer 411 with a slit, providing the lower absorbent layer 412 with a fiber-free region (a through-hole having an oblong rectangular plan-view shape), and stacking them with the slit and the fiber-free region aligned with each other or may be formed by providing the upper absorbent layer 411 with a fiber-free region, providing the lower absorbent layer 412 with a slit, and stacking them with the fiber-free region and the slit aligned with each other. As used herein, the term "slit" denotes a fiber-free region and through-hole with a relatively small width.

A groove as the central bending-inducing region 43 is exemplified by a groove formed by stacking an upper absorbent layer 411 having a through-hole of an oblong rectangular plan-view shape atop of a lower absorbent layer 412 having no through-hole or a groove formed by embossing a single-layered absorbent core formed of a fiber aggregate containing or not containing a functional material. The embossing is preferably heat embossing, ultrasonic embossing, or high frequency embossing.

As shown in Fig. 2, the absorbent member 4 in the diaper 1 of the present example has, in its absorbent core 40 in the crotch portion C, a region R1 wherein the central bending-inducing region 43 and the pair of side bending-inducing regions 44, 44 are in juxtaposition to the longitudinal direction of the absorbent core 40 (hereinafter also called "juxtaposition region R1").

The central bending-inducing region 43 extends beyond the front end 44a of each side bending-inducing region 44 toward the front end of the absorbent core 40 (toward the front end of the front portion A), and the side bending-inducing regions 44, 44 both extend beyond the rear end 43b of the central bending-inducing region 43 toward the rear end of the absorbent core 40 (toward the rear end of the rear portion B).

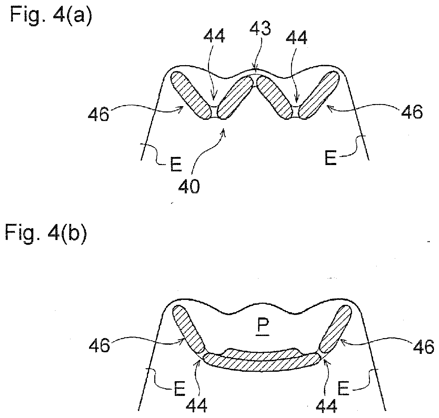

Since the absorbent core 40 has the juxtaposition region R1 in the crotch portion C, the part of the absorbent core 40 located in the juxtaposition region R1 bends into a "W" configuration when worn, with the central bending-inducing region 43 as the top of a mountain and each side bending-inducing region 44, 44 as the bottom of a valley, as shown in Fig. 4(a).

Since the absorbent core 40 is bendable into a W-configuration in the crotch portion C, the absorbent core 40 can flexibly change its shape in response to the pressure applied by the wearer's legs E so that the pressure applied to the legs and the resultant feel of resistance, discomfort, or the like felt by the legs will be reduced. Moreover, side portions 46 of the absorbent core 40 outboard of the side bending-inducing regions 44 rise to bring their distal edge along the wearer's skin, preferably the groins, thereby to provide good protection against leakage.

The part of the absorbent assembly 5 having the central bending-inducing region 43 easily comes into contact with the point of urine discharge of a wearer so that urine is smoothly wicked into the absorbent member 4 or the absorbent core 40. As a result, inconveniences will be prevented from occurring, such as urine's running on the skin or the topsheet and leaking from the front side of the diaper or urine's running rearward and being mixed with feces to cause the feces to stick to the skin.

The central bending-inducing region 43 formed in the high basis weight region 41 extends beyond the front end 44a of each side bending-inducing region 44 toward the front end of the absorbent core 40. This configuration allows the urine having been acquired by the absorbent member 4 or the absorbent core 40 in the juxtaposition region R1 or the front-side vicinity thereof to spread toward the front portion A, so that the absorption capacity of the part of the absorbent core 40 located in the front of the juxtaposition region R1 will be utilized effectively. As a result, the amount of urine absorbable by the diaper 1 without leakage increases, and urine leakage hardly occurs even during long-time use of the diaper or with a large amount of urination. Since the urine spreads as captured by the absorbent member 4 or the absorbent core 40, the problems due to the contact of the skin with urine, such as an uncomfortable wetness sensation and diaper rash, will be reduced.

The side bending-inducing regions 44 serve to raise the side portions 46 of the absorbent core 40 outboard of the side bending-inducing regions 44 so as to cause their distal edge to approach the wearer's skin, preferably the wearer's groins, as shown in Figs. 4(a) and 4(b). Nevertheless, if the side bending-inducing regions 44 excessively extend forward, the side portions of the absorbent core 40 do not easily and neatly follow the contour of the wearer's skin or groins. In the diaper of the invention, since the central bending-inducing region 43 extends forward beyond the front end 44a of each side bending-inducing region 44, the side portions 46 of the absorbent core 40 are able to neatly follow the contour of the wearer's skin or groins, thereby providing a good appearance, improved wearer comfort, and excellent leakage protection.

To further ensure the above described effects, it is preferred that the length L1 (see Fig. 2) of the juxtaposition region R1 in the longitudinal direction of the absorbent core 40 be in the range of from 20% to 80%, more preferably 20% to 60%, even more preferably 20% to 50%, of the length Lc (see Fig. 2) of the crotch portion C in the diaper longitudinal direction.

From the same standpoint, it is preferred that the central bending-inducing region 43 extend beyond the front end 44a of each side bending-inducing region 44 by a distance L2 (see Fig. 2) of 5 to 40 cm, more preferably 10 to 30 cm. With respect to the relation to the length Lc of the crotch portion C, the distance L2 is preferably 2% to 15%, more preferably 3% to 12%, of the length Lc of the crotch portion C.

The central bending-inducing region 43 may extend to have its front end 43a reaching the front portion A, but is preferably short of the front portion A in view of appearance while in use and wearer comfort. From the same viewpoint, the front end 43a of the central bending-inducing region 43 is preferably short of the front portion A by a distance L3 (see Fig. 2) of 5 cm or more, more preferably 10 to 20 cm. From the same viewpoint, the distance L4 (see Fig. 2) from the waist opening edge 7a of the front portion A to the front end 43a of the central bending-inducing region 43 is preferably 10% to 40%, more preferably 15% to 35%, of the total length L (see Fig. 2) of the diaper.

The distance L5 (see Fig. 2) between the front end 44a of each side bending-inducing region 44 and the front portion A is preferably 5% to 20%, more preferably 10% to 15%, of the length Lc (see Fig. 2) of the crotch portion C.

As stated, the side bending-inducing regions 44 each extend beyond the rear end 43b of the central bending-inducing region 43 toward the rear end of the absorbent core 40 (toward the rear end of the rear portion B). Because of this configuration, the part of the absorbent core 40 closer to the rear end of the rear portion B than the juxtaposition region R1 bends along the side bending-inducing regions 44, 44 without bending along the central bending-inducing region 43 as shown in Fig. 4(b) while the disper is worn. As a result, the absorbent core 40 forms a dished shape concave toward the skin around the exit point of feces (i.e., the anus), which is rearward of the juxtaposition region R1, whereby there is formed a pocket P concave up and capable of holding discharged feces between the wearer's skin and the absorbent assembly. Discharged urine, on the other hand, is quickly wicked into the absorbent member 4 or the absorbent core 40 and led toward the front by the configuration of the juxtaposition region R1 and the central bending-inducing region 43 extending beyond the juxtaposition region R1 toward the front. Therefore, urine is less liable to mix with feces.

Thus, inconveniences due to feces' adhering to the skin, such as wearer discomfort and the troublesome work to wipe off the fecal material from the skin surface, are avoided or greatly reduced. Excellent protection against feces leakage is also provided.

To further ensure the effects discussed above, each side bending-inducing region 44 preferably extends beyond the rear end 43b of the central bending-inducing region 43 by a distance L6 (see Fig. 2) of 20 to 120 cm, more preferably 40 to 100 cm. In relation to the length Lc of the crotch portion, the distance L6 is preferably 7% to 43%, more preferably 14% to 36%, of the length Lc.

Each side bending-inducing region 44 may extend to have its rear end 44b reaching the rear portion B but is preferably short of the rear portion B so that the side portions 46 of the absorbent core 40 may neatly follow the contour of the wearer's skin or groins.

In order to cause the absorbent core 40 to form a dish-shaped pocket P capable of holding discharged feces in the vicinity of the exit point of feces, it is preferred for each side bending-inducing region 44 to extend rearward beyond the position of the wearer's exit point of feces (i.e., the anus). It is preferred for the central bending-inducing region 43, on the other hand, not to extend rearward beyond the position of the wearer's exit point of feces (the anus). The rear end 43b of the central bending-inducing region 43 is preferably located forward of the position of the exit point of feces.

For the same purpose, the distance L7 (see Fig. 2) from the rear portion B to the rear end 43b of the central bending-inducing region 43 is preferably 80 to 150 cm, more preferably 100 to 130 cm. For the same purpose, the distance L8 (see Fig. 2) from the waist opening edge 7b of the rear portion B to the rear end 43b of the central bending-inducing region 43 is preferably 30% to 60%, more preferably 40% to 50%, of the total length L (see Fig. 2) of the diaper.

The distance L9 (see Fig. 2) from the rear portion B to the rear end 44b of each side bending-inducing region 44 is preferably 5% to 25%, more preferably 10% to 20%, of the length Lc (see Fig. 2) of the crotch portion C.

In order to further ensure one or more of the above described effects, it is preferred that the distance W1 (see Fig. 3) from the lateral side edge of the absorbent core 40 to the distal edge of the corresponding side bending-inducing region 44 be 10% to 25%, more preferably 15% to 23%, of the width W2 (see Fig. 3) of the absorbent core 40. The width W3 (see Fig. 3) of the high basis weight region 41 is preferably 20% to 50%, more preferably 25% to 45%, of the width W2 of the absorbent core 40. The width W4 of the fiber-free region providing the central bending-inducing region 43 is preferably 5% to 30%, more preferably 5% to 25%, of the width W3 of the high basis weight region 41, and is preferably 1 to 20 mm, more preferably 2 to 10 mm. The fiber-free region with a width W4 of less than 2 mm can be regarded as a slit. It is preferred that each of the above recited dimensions and dimensional relations of the regions be satisfied in at least the longitudinally central part of the crotch portion C, more preferably within 5 cm from the longitudinal center in the longitudinal direction of the absorbent core.

As shown in Figs. 2 and 3, the diaper 1 of the present embodiment has an elastic member 9 disposed along each lateral side edge of the absorbent core 40 so as to raise the side portions 46 of the absorbent core 40. The elastic member 9 is disposed in its stretched state along the interior and/or the exterior side of the core wrap sheet 45 covering the absorbent core 40 and fixed at both ends thereof in the front portion A and the rear portion B.

A pair of anti-leakage cuffs 6, 6 are provided along both lateral sides of the absorbent assembly 5. Each anti-leakage cuff 6 includes a cuff-forming sheet 60 bonded to the absorbent assembly 5, cuff-forming elastic members 61 fixed in their stretched state near along the free edge of the cuff-forming sheet 60, and a midway elastic member 62 fixed in its stretched state between the fixed edge and the free edge of the cuff-forming sheet 60.

The cuff-forming sheet 60 used in the embodiment shown in Fig. 2 is a two-ply sheet formed by folding an oblong water-repellent sheet with a prescribed width in half lengthwise, the facing two plies being bonded to each other with a hot melt adhesive, partial heat or ultrasonic sealing, or a like means. The elastic members 61 and 62 are fixed in their stretched state between the two plies.

The topsheet 2 covers the skin facing side of the absorbent core 40, with each side portion 21 thereof being fixed to and between the backsheet 3 and the cuff-forming sheet 60 on the non-skin-facing side of the absorbent core 40 by a known bonding means, such as heat sealing or an adhesive.

As shown in Figs. 2 and 3, the outer cover 10 is composed of two outer cover-forming sheets 11, 12 and elastic members for specific parts fixed in their stretched state between these two sheets. Specifically, there are waist elastic members 71 forming waist gathers along the waist opening 7, leg elastic members 81 forming leg gathers along the leg openings 8, and below-waist elastic members 91 forming below-waist gathers on two spaced apart lateral sides of a below-waist portion D (the region between a position 20 mm below the edge of the waist opening 7 and the upper end of each leg opening 8) fixedly bonded in their stretched state between the two outer cover-forming sheets 11, 12 by any bonding means, such as a hot melt adhesive.

The outer cover-forming sheets 11 and 12 are each formed of nonwoven fabric. At least one of these sheets is folded back along the circumferential edge of the waist opening 7, and the folded-back panel is fixed to the skin facing side of the front and the rear end portion of the absorbent assembly 5.

In order for the absorbent core 40 to bend into a W-configuration in the juxtaposition region R1 as shown in Fig. 4(a) and into a dish-shaped configuration in the region rearward of the juxtaposition region R1 as shown in Fig. 4(b), it is preferred that elastic members effective in pulling up or pushing up the side portions 46 of the absorbent core 40 be disposed along, e.g., both sides of the absorbent assembly 5 and the anti-leakage cuffs 6 provided to overlap both side portions of the absorbent assembly 5, like the elastic members 9, 61, and 62.

It is to be noted that providing the elastic members along the longitudinal direction of the absorbent core 40 is not essential because, even if there are no elastic members along the longitudinal direction of the absorbent core 40, the absorbent core 40 is deformable into the shape shown in Figs. 4(a) or 4(b) by a vertical force of grasping and pulling up the laterally middle part of the front portion A when the diaper is fitted onto a wearer or a sideways force applied by the wearer's thighs to the crotch portion C while the disper is worn.

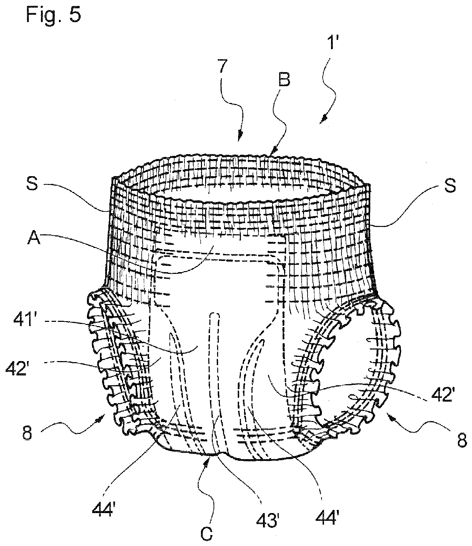

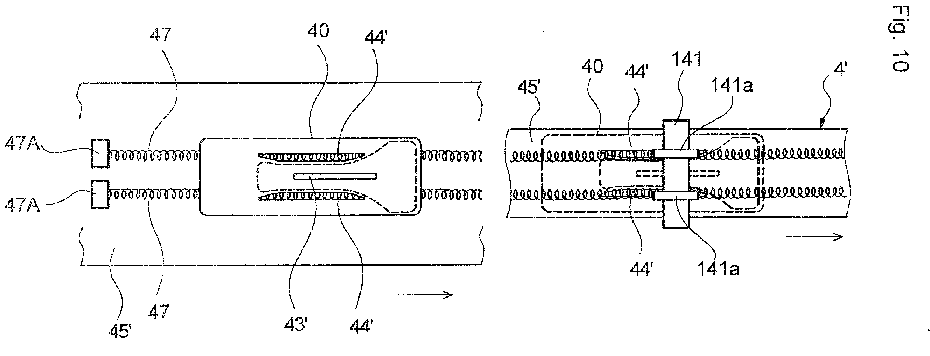

A disposable diaper 1' according to a first embodiment of the invention (hereinafter "diaper 1′ʺ) will then be described by way of Figs. 5 through 10. The description of the diaper 1' of the first embodiment will be largely confined to the differences from the diaper 1 already discussed. For the rest, the description of the diaper 1 including the preferred mode will be applied as appropriate.

The diaper 1' of the first embodiment is of pull-on type. As shown in Figs. 5 through 7, the disper 1' includes an absorbent assembly 5 and an outer cover 10 located on the non-skin-facing side of the absorbent assembly 5 and having the absorbent assembly 5 fixed thereto. The absorbent assembly 5 includes a liquid permeable topsheet 2, a liquid impermeable or water repellent backsheet 3, and an oblong absorbent member 4' interposed between the sheets 2 and 3.

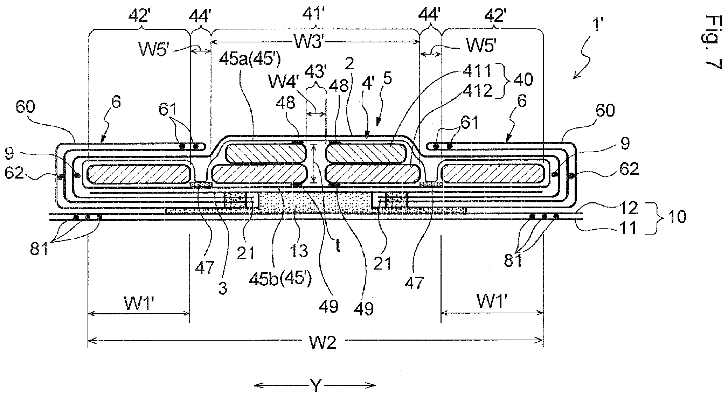

The absorbent member 4' includes an oblong absorbent core 40 containing an absorbent polymer and a core wrap sheet 45' covering the upper and the lower surface of the absorbent core. As shown in Fig. 7, the core wrap sheet 45' wraps the entire transverse cross-sectional circumference of the absorbent core 40 including the upper and the lower surface and both lateral side faces of the absorbent core 40.

As used for a disposable diaper and an absorbent core thereof, the term "the skin-facing side" or "the upper side" means the side facing the skin of a wearer while the diaper is worn, and the term "the non-skin-facing side" or "the lower side" means the side facing opposite to the wearer's skin while the diaper is worn.

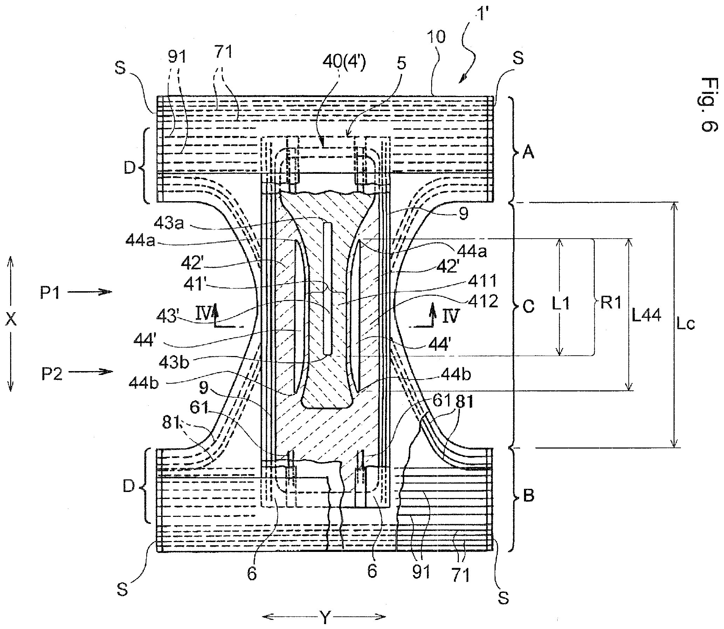

As shown in Fig. 5 and 6, the diaper 1' has a front portion A, a crotch portion B, and a rear portion C, adapted to be worn about the front side, the crotch, and the rear side, respectively, of a wearer. The outer cover 10 has its opposite side edges in the front portion A joined to those in the rear portion B to form a pair of side seals S, S, a waist opening 7, and a pair of leg openings 8, 8.

The front portion A and the rear portion B of the disposable pull-on diaper are each defined to be a portion having the side seal S along both lateral side edges (opposite in the direction Y in Fig. 6). The longitudinal direction of the diaper is the direction extending from the front portion A through the crotch portion C to the rear portion B or the opposite direction, which is the direction X in Fig. 6. In the diaper 1' of the present embodiment, all of the longitudinal direction of the diaper 1', the longitudinal direction of the absorbent member 4', the longitudinal direction of the absorbent core 40, and the longitudinal direction of a hereinafter described central absorbent portion 41' are the direction X in Fig. 6.

As shown in Fig. 6 and 7, the absorbent core 40 in the diaper 1' has, in the crotch portion C, a central absorbent portion 41' extending in the longitudinal direction of the absorbent core 40 and a pair of side absorbent portions 42' located on both sides of central absorbent portion 41'. In detail, as shown in Fig. 6, the absorbent core 40 is composed of an upper absorbent layer 411 having a generally T-shape in a plan view with an increased width in its front portion side and a lower absorbent layer 412 larger than the upper absorbent layer 411 in both the longitudinal and the transverse direction.

As shown in Fig. 7, the lower absorbent layer 412 has a pair of through-holes as side fiber-free regions 44', 44'. The side absorbent portions 42', 42' are portions located laterally (in the direction Y) outward from the fiber-free regions 44', 44', and the central absorbent portion 41' is a portion located between the side fiber-free regions 44', 44'.

The upper absorbent layer 411 extends through the crotch portion C and terminates in the front portion A. The lower absorbent layer 412 extends through the crotch portion C and terminates in the front portion A and the rear portion B.

As shown in Figs. 6 and 7, the absorbent core 40 of the diaper 1' has a central fiber-free region 43', where the absorbent core is cut out throughout the thickness, in the laterally middle part of the central absorbent portion 41' as well as the side fiber-free regions 44', 44' between the central absorbent portion 41' and both side absorbent portions 42', 42'. Each side fiber-free region 44' is located between the corresponding lateral side edge of the upper absorbent layer 411 and the lateral side edge of the absorbent core 40 and closer to the upper absorbent layer 411 than to the side edge of the absorbent core 40. Each side fiber-free region 44' extends along each side edge of the central absorbent portion 41'.

In the diaper 1' of the first embodiment, the central fiber-free region 43' functions as the central bending-inducing region of the invention, and the side fiber-free region 44' functions as the side bending-inducing region of the invention.

The upper absorbent layer 411 and the lower absorbent layer 412 making up the absorbent core 40 of the present embodiment are each formed of a fiber aggregate made of a fibrous material, such as defibrated pulp, with or without a particulate absorbent polymer incorporated therein. The absorbent member 4' is formed by totally covering the upper absorbent layer 411 and the lower absorbent layer 412 thus configured with a core wrap sheet 45. The core wrap sheet may be of various known materials, preferably thin paper (e.g., tissue) or water pervious nonwoven fabric.

The side fiber-free region 44' is a region having no materials forming the absorbent core (core-forming material). As shown in Fig. 7, the upper and the lower panel of the core wrap sheet covering the absorbent core 40 are bonded to each other in each side fiber-free region 44'. Although it is preferred that there be no core-forming material at all in the side fiber-free region 44' so that the core wrap sheet panels may firmly be bonded, presence of a small amount of a core-forming material is acceptable as long as the direct bonding of the core wrap sheet panels with an adhesive is not impeded.

On the other hand, the central fiber-free region 43' is a region of the central absorbent portion 41' where at least part of the upper side thereof is cut out. The central fiber-free region 43' preferably has a depth equal to or greater than the thickness of the upper absorbent layer, more preferably a depth substantially equal to the thickness t of the central absorbent portion 41' at a position adjacent to the central fiber-free region 43' as shown in Fig. 7.

As shown in Fig. 7, the central fiber-free region 43' in the present embodiment is a through-hole piercing the thicknesses of both the upper and the lower absorbent layer 411 and 412. The through-hole piercing the upper and the lower absorbent layer 411 and 412 may be formed by making an upper and a lower absorbent layer each having a through-hole and stacking them with their through-holes aligned with each other; by making an upper and a lower absorbent layer each having no through-hole, cutting a through-hole in each of them, and stacking them with their through-holes aligned with each other; or by making an upper and a lower absorbent layer each having no through-hole, stacking them, and cutting a through-hole piercing the stacked layers.

In the present embodiment, the core wrap sheet panel 45a overlying the absorbent core 40 is pressed into each side fiber-free region 44', and the pressed-in part of the panel 45a is bonded to the core wrap sheet panel 45b underlying the absorbent core 40 via an adhesive 47. The adhesive 47 may be applied either continuously or discontinuously in the longitudinal direction of the side fiber-free region 44'. While the adhesive 47 may be applied solid to the entire adherend area corresponding to the side fiber-free region 44', it is preferably applied in a regular pattern over the entire area extending in the longitudinal direction of the side fiber-free region 44'. For example, the adhesive 47 may be applied to the core wrap sheet panel 45a or 45b in a pattern, such as a spiral, a continuous wave, a zig-zag, or a continuous omega pattern, with a width equal to or larger than the width of the side fiber-free region 44' along the longitudinal direction of the absorbent core 40. The absorbent core 40 is placed on or under the core wrap sheet with its side fiber-free regions 44' in register with the applied adhesive 47, and the upper and the lower core wrap sheet panels are bonded to each other via the adhesive. The width of the applied adhesive may be smaller than the width of the side fiber-free region 44'. For example, the adhesive may be applied to form spaced away adhesive layers within the width of the side fiber-free region 44' each extending in the longitudinal direction of the side fiber-free region 44'. The adhesive 47 is preferably applied over a length of at least 50%, more preferably 80% or more, of the whole length L44 of the side fiber-free region 44' and over the whole length of the juxtaposition region R1, more preferably over the whole length of the side fiber-free region 44'.

On the other hand, the part of the core wrap sheet panel 45a overlying the absorbent core 40 and corresponding to the central fiber-free region 43' is not bonded to the member lying right under that part of the core wrap sheet panel 45a, i.e., the lower core wrap sheet panel 45b. It is preferred that the adhesive be absent on the lower side (the absorbent core side) of the part of the core wrap sheet panel 45a corresponding to the central fiber-free region 43', but the adhesive may be present on that part. Even when there is an adhesive on that part of the upper core wrap sheet panel 45a, a space in which the absorbent polymer or the absorbent core 40 containing the absorbent polymer is allowed to swell upon fluid absorption would be secured unless the adhesive causes adhesion to the part of the member located right thereunder.

When a means is taken to prevent the adhesive from contacting the member located right thereunder, a space allowing the absorbent polymer or the absorbent core 40 containing the absorbent polymer to swell upon fluid absorption will be secured more certainly between the lower side of the core wrap sheet panel 45a and the member located right thereunder.

As will be described with respect to a preferred method of production, such a means is exemplified by applying a tension to the core wrap sheet panel 45a between the two side fiber-free regions 44', 44' when bonding the upper and the lower core wrap sheet panels 45a and 45b to each other in the side fiber-free regions 44', 44', the tension being applied by using a pressure roller 141 having a pressing projection at the position corresponding to each side fiber-free region 44' and no pressing projection at the position corresponding to the central fiber-free region 43'. In the case where an adhesive (e.g., a pressure-sensitive adhesive) is applied as well to the part of the core wrap sheet panel 45a corresponding to the central fiber-free region 43' or, when the central fiber-free region 43' is a through-hole, to the part of the core wrap sheet panel 45b corresponding to the central fiber-free region 43', it is desirable that the width W4 of the central fiber-free region 43' is 20 mm or less, more desirably 12 mm or less, so as to prevent the bonding via the adhesive.

It is preferred that the absorbent core 40 be fixed to the underlying core wrap sheet panel 45b in other than the regions corresponding to the central fiber-free region 43' and the side fiber-free regions 44', 44' with an adhesive (not shown) applied in such a regular pattern as recited above, i.e., a spiral, a continuous wave, a zig-zag, or a continuous omega pattern.

In the embodiment shown in Fig. 7, the core wrap sheet panel 45a overlying the absorbent core 40 is bonded via an adhesive 48 to the absorbent core 40 at positions oppositely neighboring the central fiber-free region 43' in the transverse direction thereof. Similarly, the core wrap sheet panel 45b underlying the absorbent core 40 is bonded via an adhesive 49 to the absorbent core 40 at opposite positions neighboring the central fiber-free region 43' in the transverse direction thereof. Bonding the absorbent core 40 to the core wrap sheet panels 45a and/or 45b at positions oppositely neighboring the central fiber-free region 43' in the transverse direction thereof is effective in preventing every portion of the absorbent core 40 from losing its shape or from deviating from the positional relationship among portions as designed, thereby to secure a space for allowing the absorbent polymer or the absorbent core 40 containing the absorbent polymer to swell upon fluid absorption more certainly. The adhesive 48 and the adhesive 49 are each preferably applied in a regular pattern in the longitudinal direction of each side fiber-free region 44'. The adhesive 48 and the adhesive 49 are preferably applied over a length of at least 50%, more preferably 80% or more, of the whole length L44 of the side fiber-free region 44' and over the whole length of the juxtaposition region R1, more preferably over the whole length of the side fiber-free region 44'. The adhesive 48 and 49 may be applied either solid or patternwise in such a pattern as recited as for the adhesive 47.

According to the configuration of the diaper 1' of the first embodiment, the central fiber-free region 43' provides a space defined by the absorbent core 40 and the overlying core wrap sheet panel 45a, and the core wrap sheet panel 45a on the central fiber-free region 43' is not bonded to the member located right thereunder (the core wrap sheet panel 45b). Therefore, when a fluid is wicked into the central absorbent portion 41' to cause the absorbent polymer present therein to swell, the most of the volumetric increase of the absorbent polymer is absorbed by that space, so that the absorbent polymer is allowed to swell sufficiently without any hindrance.

As a result, the absorptivity of the absorbent polymer is developed to the full, and incorporation of the absorbent polymer effectively leads to improvement on disposable diaper performance.

The bonding of the upper and the lower core wrap sheet panels 45a and 45b overlying and underlying, respectively, of the absorbent core in the side fiber-free regions 44' guarantees excellent shape retention of the absorbent core 40. That is, the central absorbent portion 41' and the side absorbent portions 42', 42' are prevented from losing their shape or being misaligned with each other while the diaper is worn.

As earlier described, the central absorbent portion 41' allows the absorbent polymer contained therein to fully exert its absorptivity by virtue of the central fiber-free region 43'. This being taken advantage of, it is preferred that absorbent polymer be incorporated in a higher basis weight (amount per unit area) in the central absorbent portion 41' than in the side absorbent portions 42', 42' to achieve efficient improvement on the performance of the disposable diaper expected of the incorporation of an absorbent polymer. From the same viewpoint, in the case where the central absorbent portion 41' has a multi-layered (or dual-layered) structure having the upper absorbent layer 411 and the lower absorbent layer 412, the absorbent polymer is preferably incorporated in the upper absorbent layer 411 in a higher basis weight than in the lower absorbent layer 412. In the case of a multi-layered structure having three or more layers, the absorbent polymer is preferably incorporated into the uppermost absorbent layer in a higher basis weight than any other absorbent layer.

The central fiber-free region 43' according to the first embodiment also functions to accelerate spread of a fluid in the longitudinal direction of the absorbent core 40 and to improve transverse bending properties of the absorbent core 40.

The side fiber-free regions 44' according to the first embodiment function to accelerate spread of a fluid in the longitudinal direction of the absorbent core and to improve transverse bending properties of the absorbent core 40.

As shown in Fig. 6, the absorbent member 4' in the diaper 1' of the first embodiment has, in its absorbent core 40 in the crotch portion, a region R1 wherein the central fiber-free region 43' and the pair of side fiber-free regions 44', 44' are in juxtaposition in the transverse direction of the absorbent core 40 (hereinafter also called "juxtaposition region R1").

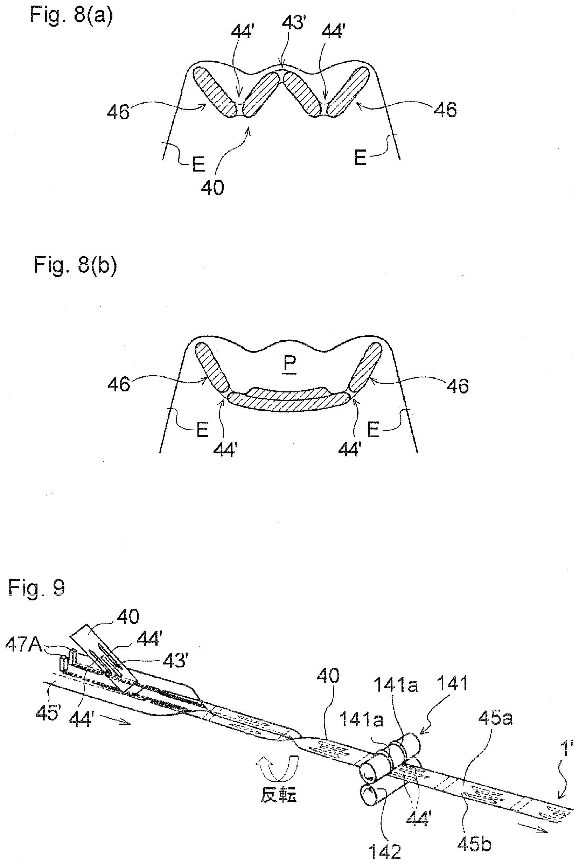

Because of the juxtaposition region R1 in the crotch portion C, the part of the absorbent core 40 located in the juxtaposition region R1 bends into a "W" configuration when worn, with the central fiber-free region 43' as the top of a mountain and each side fiber-free region 44' as the bottom of a valley, as shown in Fig. 8(a).

Since the absorbent core 40 is bendable into a W-configuration in the crotch portion C, the absorbent core 40 can flexibly change its shape in response to the pressure applied by the wearer's legs E so that the pressure applied to the legs and the resultant resistance, discomfort, or the like felt by the legs will be reduced. Moreover, side portions 46 of the absorbent core 40 outboard of the side fiber-free regions 44' rise to have their distal edge located along the wearer's skin, preferably the groins, thereby to provide good protection against leakage.

The part of the absorbent assembly 5 having the central fiber-free region 43' easily comes into contact with the wearer's exit point of urine so that urine is smoothly wicked into the absorbent member 4 or the absorbent core 40. The urine having been acquired by the absorbent member 4 or the absorbent core 40 spreads toward the front and the rear so that the absorption capacity of the portions of the absorbent core 40 located in the front and the rear of the juxtaposition region R1 will be utilized effectively as well. As a result, the amount of urine absorbable by the diaper 1 without leakage increases, and urine leakage hardly occurs even during long-time use of the diaper or with a large amount of urination. Since the urine spreads as captured by the absorbent member 4 or the absorbent core 40, the problems due to the contact of the skin with urine, such as an uncomfortable wetness sensation and diaper rash, will be reduced.

The part of the absorbent core 40 closer to the rear end of the rear portion B than the juxtaposition region R1 bends along the side fiber-free regions 44', 44' as shown in Fig. 8(b) while the disper is worn. As a result, the absorbent core 40 forms a dished shape concave toward the skin around the exit point of feces (i.e., the anus), whereby there is formed a pocket P concave up and capable of holding discharged feces between the wearer's skin and the absorbent assembly. Thus, inconveniences due to feces' adhering the skin, such as wearer discomfort and the troublesome work to wipe off the fecal material from the skin surface, are avoided or greatly reduced. Excellent protection against feces leakage is also provided.

To further ensure one or more of the above described effects, it is preferred that the length L1 (see Fig. 6) of the juxtaposition region R1 in the longitudinal direction of the absorbent core 40 be in the range of from 20% to 80%, more preferably 20% to 60%, even more preferably 20% to 50%, of the length Lc (see Fig. 6) of the crotch portion C in the diaper longitudinal direction.

It is preferred that the width W1' (see Fig. 7) of the side absorbent portion 42' be 5% to 30%, more preferably 10% to 25%, of the width W2 (see Fig. 7) of the absorbent core 40. The width W3' (see Fig. 7) of the central absorbent portion 41' is preferably 20% to 70%, more preferably 25% to 50%, of the width W2 of the absorbent core 40. The width W4' of the central fiber-free region 43' is preferably 1 to 25 mm, more preferably 2 to 15 mm, and is preferably, in terms of relation to the width W3' of the central absorbent portion 41', 3% to 30%, more preferably 5% to 25%, of W3' of the central absorbent portion 41'. The width W5' of the side fiber-free region 44' is preferably 1 to 35 mm, more preferably 5 to 25 mm, and is preferably, in terms of relation to the width W2 of the absorbent core 40, 1% to 30%, more preferably 5% to 20%, of W2 of the absorbent core 40.

As shown in Figs. 6 to 7, the diaper 1' of the present embodiment has an elastic member 9 disposed along each lateral side edge of the absorbent core 40 to raise the side portions 46 of the absorbent core 40. The elastic member 9 is disposed in its stretched state along the interior and/or the exterior side of the core wrap sheet 45 covering the absorbent core 40 and fixed at both ends thereof in the front portion A and the rear portion B.

A pair of anti-leakage cuffs 6, 6 are formed along both lateral sides of the absorbent assembly 5. Each anti-leakage cuff 6 includes a cuff-forming sheet 60 bonded to the absorbent assembly 5, cuff-forming elastic members 61 fixed in their stretched state near along the free edge of the cuff-forming sheet 60, and a midway elastic member 62 fixed in its stretched state between the fixed edge and the free edge of the cuff-forming sheet 60.

The cuff-forming sheet 60 used in the embodiment shown in Fig. 7 is a two-ply sheet formed by folding an oblong water-repellent sheet with a prescribed width in half lengthwise, the facing two plies being bonded to each other with a hot melt adhesive, partial heat or ultrasonic sealing, or a like means. The elastic members 61 and 62 are fixed in their stretched state between the two plies.

The topsheet 2 covers the upper side (skin facing side) of the absorbent core 40, with each side portions 21 thereof being fixed to the backsheet 3 and the cuff-forming sheet 60 on the lower side (non-skin-facing side) of the absorbent core 40 by a known bonding means, such as heat sealing or an adhesive. The absorbent assembly 5 is bonded to the outer cover 10 with an adhesive 13 in its laterally middle portion.

As shown in Figs. 6 and 7, the outer cover 10 is composed of two outer cover-forming sheets 11, 12 and elastic members for specific parts fixed in their stretched state between these two sheets. Specifically, there are waist elastic members 71 forming waist gathers along the waist opening 7, leg elastic members 81 forming leg gathers along the leg openings 8, and below-waist elastic members 91 forming below-waist gathers on two spaced apart lateral sides of a below-waist portion D (the region between a position 20 mm below the edge of the waist opening 7 and the upper end of each leg opening 8) fixedly bonded in their stretched state between the two outer cover-forming sheets 11, 12 by any bonding means, such as a hot melt adhesive.

The outer cover-forming sheets 11 and 12 are each formed of nonwoven fabric. At least one of these sheets is folded back along the edge of the waist opening 7, and the folded-back panel is fixed to the skin facing side of the front and the rear end portion of the absorbent assembly 5.

In order for the absorbent core 40 to bend into a W-configuration in the juxtaposition region R1 as shown in Fig. 8(a) and into a dish-shaped configuration in the region rearward of the juxtaposition region R1 as shown in Fig. 8(b), it is preferred that elastic members effective in pulling up or pushing up the side portions 46 of the absorbent core 40 be disposed along both sides of the absorbent assembly 5, the anti-leakage cuffs 6 provided to overlap both side portions of the absorbent assembly 5, and so on like the elastic members 9, 61, and 62.

It is to be noted that, even if there are no elastic members disposed along the longitudinal direction of the absorbent core 40, the absorbent core 40 is deformable into the shape shown in Figs. 8(a) or 8(b) by a vertical force of grasping and pulling up the laterally middle part of the front portion A when the diaper is fitted onto a wearer or a sideways force applied by the wearer's thighs to the crotch portion C while the disper is worn.

Materials making up the diapers 1 and 1' will then be described.

Any various materials that have been used in each member of conventional disposable diapers may be used to make the corresponding member of the diapers 1 and 1'. For example, the topsheet 2 may be formed of water-wettable and liquid permeable nonwoven fabric or perforated resin film, and the backsheet 3 may be formed of moisture permeable or impermeable resin film or a laminate of such resin film and nonwoven fabric.

Fibers constituting the fiber aggregate forming the absorbent core 40 include hydrophilic fibers, such as pulp fiber, rayon fiber, cotton fiber, and cellulose acetate fiber, polyolefin fibers, such as polyethylene and polypropylene, and polycondensation fibers, such as polyesters and polyamides. The fiber aggregate is preferably an airlaid fiber aggregate prepared by accumulating a fibrous material carried in an air stream, either alone or as mixed with a functional material, in a recess of prescribed shape by suction. A functional material, if used, may be held between two fiber aggregates or among fibers in a mixed airlaid fiber aggregate. Two or more kinds of fibers or two or more functional materials, such as absorbent polymers, may be used in combination. Examples of the particulate absorbent polymer include sodium polyacrylate, acrylic acid-vinyl alcohol copolymers, crosslinked sodium polyacrylate, a starch-acrylic acid graft copolymer, an isobutylene-maleic anhydride copolymer and a saponification product thereof, and polyaspartic acid.

The anti-leakage cuff-forming sheet 60 may be, for example, composite nonwoven fabric having a multi-layer structure (e.g., spun-bonded/melt-blown/spun-bonded), spun-bonded nonwoven fabric, heat-bonded nonwoven fabric, or air-through nonwoven fabric. The elastic member to be disposed in various parts or members is preferably an elastic thread (e.g., rubber thread) or an elastic tape (e.g., rubber tape), particularly an elastic thread. Materials of the elastic member to be disposed in various parts or members include natural rubber, synthetic rubbers, e.g., styrene-butadiene rubber, butadiene rubber, isoprene rubber, and neoprene rubber, EVA, stretch polyolefins, and urethane rubber.

The diaper 1' is preferably produced by a method described below.

The method includes the steps of making an absorbent core 40 composed of a central absorbent portion 41' having a central fiber-free region 43' and a pair of side absorbent portions 42', 42' having respective side fiber-free regions 44' along the central absorbent portion 41' (unshown step), applying an adhesive 47 to parts of a core wrap sheet 45' corresponding to the side fiber-free regions 44', and pressing the absorbent core 40 having the core wrap sheet 45' on the upper and the lower side thereof using a pressure roller 141 having a pressing projection at the position corresponding to each side fiber-free region 44' with no pressing projection at the position corresponding to the central fiber-free region 43', thereby to press one or both of the upper and the lower panels of the core wrap sheet 45' into the side fiber-free regions 44' to bond the upper and the lower core wrap sheet panels 45a and 45b to each other.

The absorbent core 40 is preferably made using a fiber airlaying apparatus having, for example, two airlaying drums each having a recess of prescribed shape in which a fibrous material transported by air is deposited by suction. A lower absorbent layer 412 having through-holes providing a central fiber-free region 43' and side fiber-free regions 44', 44' is formed on one of the airlaying drums, and an upper absorbent layer 411 having a through-hole providing the central fiber-free region 43' is formed on the other airlaying drum. The absorbent layers 411 and 412 are stacked to make the absorbent core 40 having a through-hole piercing the two absorbent layers as a central fiber-free region 43'. The adhesive 47 can be applied to the parts of the core wrap sheet 45' corresponding to the side fiber-free regions 44' by the use of any known adhesive applicator 47A.

Bonding the upper and the lower core wrap sheet panels 45a and 45b at the side fiber-free regions 44' is achieved by pressing using a combination of the above described pressure roller 141 and either one of a smooth roller 42' and a second roller 42' having similar projections at the positions corresponding to the pressing portions 141a of the pressure roller 141. The projections as the pressing portions 141a may be continuous around the entire circumference of the pressure roller 141 or over a certain length in the circumferential direction of the pressure roller 141. The diaper 1' can be produced in the same manner as for the production of conventional disposable pull-on diapers except for the making of the absorbent member 4'. For example, the method described in JP 2006-247363A may be followed to advantage.

When the parts of the core wrap sheet panel 45a corresponding to the side fiber-free regions 44' are pressed in and bonded to the core wrap sheet panel 45b, a tension is applied to the core wrap sheet panel 45a between the two side fiber-free regions 44', 44'. As a result, a space allowing the absorbent polymer or the absorbent core containing the absorbent polymer to easily swell upon fluid absorption will be secured more certainly between the upper surface of the absorbent core 40 and the core wrap sheet panel 45a.

The diaper 1 according to the example may be produced in the same manner as for the production of the diaper 1'. In that case, the central fiber-free region 43' corresponds to the central bending-inducing region of the diaper 1, and the side fiber-free regions 44' correspond to the side bending-inducing regions of the diaper 1.

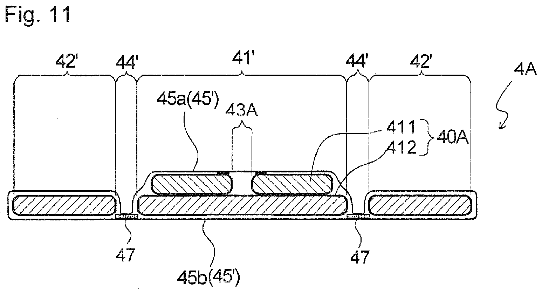

A second embodiment of the invention will then be described. Fig. 11 is a transverse cross-section of an absorbent member 4A used in a disposable diaper according to the third embodiment of the invention. The description of the second embodiment will be largely confined to the differences from the diaper 1' already discussed. For the rest, the description of the diaper 1' including the preferred mode will be applied as appropriate.

The absorbent core 40A of the absorbent member 4A shown in Fig. 11 is composed of an upper absorbent layer 411 having a through-hole providing a central fiber-free region 43'A and a lower absorbent layer 412 having no through-hole aligned with the through-hole of the upper absorbent layer 411. Like this configuration, the central fiber-free region (central bending-inducing region) as specified in the invention may be a through-hole (fiber-free region) formed through only the upper absorbent layer 411 that forms the central absorbent portion 41' together with the lower absorbent layer 412.

In the diaper of the second embodiment, the central fiber-free region 43'A functions as the central bending-inducing region as specified in the invention, and side fiber-free regions 44' hereinafter described function as side bending-inducing regions as specified in the invention. The central fiber-free region 43 as specified in the invention may be a through-hole piercing the absorbent core 40 or a groove formed on the absorbent core. The central fiber-free region 43 or the central bending-inducing region may be a slit cut through the absorbent core 40.

In the second embodiment shown in Fig. 11, too, the core wrap sheet panel 45a overlying the absorbent core 40 is pressed inside each side fiber-free region 44', and the pressed-in part is bonded to the core wrap sheet panel 45b underlying the absorbent core 40 via an adhesive 47. On the other hand, the part of the core wrap sheet panel 45a overlying the central fiber-free region 43'A of the absorbent core 40 is not bonded to the upper side of the lower absorbent layer 412 that is a member located right thereunder.

In the embodiment shown in Fig. 11, too, when a fluid is wicked into the central absorbent portion 41' to cause the absorbent polymer present therein to swell, the presence of the central fiber-free region 43'A allows the absorbent polymer to swell sufficiently without encountering a hindrance. As a result, the absorptivity of the absorbent polymer is developed to the full, and incorporation of the absorbent polymer effectively leads to improvement on disposable diaper performance. The bonding of the upper and the lower core wrap sheet panels 45a and 45b overlying and underlying, respectively, of the absorbent core guarantees excellent shape retention of the absorbent core. That is, the central absorbent portion 41' and the side absorbent portions 42', 42' are prevented from losing their shape or being misaligned with each other.

For instance, the disposable diaper of the invention may be a pull-on diaper of separate outer cover type, in which the outer cover is separated into a rear side panel adapted to be worn about the rear side of a wearer and a front side panel adapted to be worn about the front side of a wearer. The rear side panel and the front side panel are bonded along a pair of side seals to form a cylindrical shape, and the absorbent assembly is fixed to bridge the front side panel and the rear side panel. The disposable diaper of the invention may be of flat type having a fastening tape in the rear portion and a landing tape receiving the fastening tape in the outer side of the front portion. In the flat type disposable diaper, the crotch portion is a portion having a concave cutout in conformity to the contour of a wearer's leg along each lateral side edge thereof, and the rear and the front portion are portions adjacent to and forward and backward, respectively, of the crotch portion.

The diapers 1 and 1' may dispense with the elastic members 9. The elastic members 62 disposed in the anti-leakage cuffs 6, 6 and even the anti-leakage cuffs per se may be dispensed with. Instead, the absorbent assembly 5 may have an elastic member disposed along and outside each lateral side edge of its absorbent core 40 to form a pair of side gathers along each lateral side edge thereof. This elastic member can serve as an elastic member to raise the corresponding side portion of the absorbent core 40.

The absorbent assembly 5 may not have any elastic member along each lateral side edge of its absorbent core 40.

The high basis weight region may be a thickened portion provided in a part of a single-layered absorbent core. The slit, groove, or fiber-free region serving as a central bending-inducing region and the fiber-free regions serving as side bending-inducing regions do not need to be continuous but may contain a discontinuous part.

The central fiber-free region 43' and each side fiber-free region 44' are preferably configured to cause the absorbent core 40 to bend into the shapes shown in Figs. 4(a) and 4(b).

The core wrap sheet may wrap the entire transverse cross-sectional circumference of the absorbent core or may include two separate sheets, one overlying and the other underlying the absorbent core. In the case when a single core wrap sheet wraps around the absorbent core, the joint (preferably a lap joint) of the opposite edges of the sheet may be on either the upper side or the lower side but is preferably on the lower side of the absorbent core, more preferably between the opposite side fiber-free regions 44', 44'. In the case when two wrap sheets are used, it is preferred that both lateral side portions of the underlying sheet be folded over the upper side of the absorbent core and that both lateral side portions of the overlying sheet be bonded to the folded-over portions of the underlying sheet. Otherwise, it is preferred that both lateral side portions of the overlying sheet be folded over the lower side of the absorbent core and that both lateral side portions of the underlying sheet be bonded to the folded-over portions of the overlying sheet.

The central fiber-free region 43' and the pair of side fiber-free regions 44', 44' may be through-holes cut in juxtaposition through a single-layered absorbent core each in a shape that is oblong in a plan view longer in the longitudinal direction of the absorbent core. In this modification, the central absorbent portion 41' and each side absorbent portion 42' may have the same thickness, or the former may be thicker than the latter. In the case of such a single-layered absorbent member, the absorbent member and the core wrap sheet can be bonded to each other according to the embodiments shown in Figs. 7 and 11 or the preferred modes of these embodiments. By so doing, the same effects as by the diaper 1' and the other earlier stated diapers will be obtained to advantage.

In the diaper 1', the joints formed by the adhesive 48 and/or the adhesive 49 may be dispensed with.

With respect to particulars that have not been described for one embodiment, the corresponding details of other embodiments appropriately apply, and the particulars described as being characteristic of one embodiment appropriately apply to other embodiments. Particulars of each of the aforementioned embodiments are interchangeable between embodiments.

### Industrial Applicability

The invention provides a disposable diaper having excellent protection against urine and feces leakage and reducing discomfort of having feces adhering to the skin.

## Claims

1. A disposable diaper comprising an absorbent member containing an oblong absorbent core, the diaper having a front portion, a crotch portion, and a rear portion adapted to be worn about the front, the crotch, and the rear, respectively, of a wearer,
the absorbent core having a high basis weight region with a high basis weight of an absorbent core-forming material in a laterally middle part of the crotch portion and a low basis weight region having a lower basis weight of the absorbent core-forming material than the high basis weight region in both lateral sides of the high basis weight region,
the absorbent core having a central bending-inducing region extending in its longitudinal direction in the form of a slit, a groove, or a fiber-free region in the high basis weight region thereof and a side bending-inducing region in the form of a fiber-free region in each of the low basis weight regions thereof, wherein the fiber-free region is a region where any absorbent core-forming material is not present or wherein the fiber-free region is a region where an absorbent core-forming material is present in a total amount of 50 g/m² or less,
**characterized in that**
the central bending-inducing region extending in the longitudinal direction of the absorbent core toward the front end of the absorbent core beyond the front end of each side bending-inducing region,
the side bending-inducing regions extending in the longitudinal direction of the absorbent core toward the rear end of the absorbent core beyond the rear end of the central bending-inducing region,
the absorbent core containing an absorbent polymer, the absorbent member comprising the absorbent core and a core wrap sheet covering the upper and the lower side of the absorbent core,
the part of the core wrap sheet overlying the absorbent core and the part of the core wrap sheet underlying the absorbent core being bonded to each other in each side bending-inducing region, and
the part of the core wrap sheet overlying the absorbent core is not bonded to a member located right thereunder in the central bending-inducing region.

2. The disposable diaper according to claim 1, wherein the high basis weight region has a width increasing from an area where the central bending-inducing region and the side bending-inducing regions are in a juxtaposed relation toward the front end of the front portion.

3. The disposable diaper according to claim 1 or 2, wherein the front end of the central bending-inducing region is short of the front portion.

4. The disposable diaper according to any one of claims 1 to 3, wherein the rear end of each side bending-inducing region is beyond the rear end of the central bending-inducing region by a distance L6 of 20 to 120 cm.

5. The disposable diaper according to any one of claims 1 to 4, wherein the rear end of the central bending-inducing region is at or short of the position corresponding to the wearer's exit point of feces, and the rear end of each side bending-inducing region is beyond the position corresponding to the wearer's exit point of feces.

6. The disposable diaper according to any one of claims 1 to 5, wherein the absorbent core comprises an upper absorbent layer and a lower absorbent layer larger than the upper absorbent layer in both the longitudinal and the transverse direction, the high basis weight region is a region where the upper and the lower absorbent layer overlap, the low basis weight regions are each a region where the upper and the lower absorbent layer do not overlap, and the central bending-inducing region is a rectangular through-hole longer in the longitudinal direction of the absorbent core and piercing through the upper and the lower absorbent layer.

7. The disposable diaper according to claim 6, wherein the through-hole is the result of stacking the upper and the lower absorbent layers each having a through-hole with their through-holes aligned with each other.

8. The disposable diaper according to any one of claims 1 to 7, wherein the absorbent core has, in the crotch portion, a central absorbent portion extending in its longitudinal direction and a pair of side absorbent portions located on both lateral sides of the central absorbent portion, and the central absorbent portion contains the absorbent polymer in a higher basis weight than each side absorbent portion.

9. The disposable diaper according to any one of claims 1 to 8, wherein the central absorbent portion has a multi-layered structure comprising an upper absorbent layer and a lower absorbent layer, and the upper absorbent layer contains the absorbent polymer in a higher basis weight than the lower absorbent layer.

10. The disposable diaper according to any one of claims 1 to 9, being of pull-on type, comprising an absorbent assembly having the absorbent member and an outer cover located on the non-skin-facing side of the absorbent assembly and having the absorbent assembly fixed thereto, the outer cover having its opposite lateral side edges in the front portion joined to those in the rear portion to form a pair of side seals, a waist opening, and a pair of leg openings.

11. The disposable pull-on diaper according to claim 10, being of separate outer cover type wherein the outer cover is separated into a rear side panel adapted to be worn about the rear side of a wearer and a front side panel adapted to be worn about the front side of a wearer, the rear side panel and the front side panel being joined to each other along the pair of side seals to form a cylindrical shape, and the absorbent assembly being fixed to bridge the front side panel and the rear side panel.

12. The disposable diaper according to any one of claims 1 to 9, being of flat type having a fastening tape in the rear portion and a landing tape receiving the fastening tape in the outer side of the front portion.

## Patentansprüche

1. Wegwerfwindel aufweisend ein absorbierendes Element, das einen länglichen absorbierenden Kern aufweist, wobei die Windel einen vorderen Abschnitt, einen Schrittabschnitt und einen hinteren Abschnitt hat, die geeignet sind, am Körper um die Vorderseite, den Schritt bzw. die Rückseite eines Trägers getragen zu werden, wobei
der absorbierende Kern aufweist: einen Hochbasisgewicht-Bereich mit einem hohen Basisgewicht eines absorbierenden Kern bildenden Materials in einem lateral mittleren Teil des Schrittabschnitts und einen Niederbasisgewicht-Bereich mit einem niedrigeren Basisgewicht des absorbierenden Kern bildenden Materials als der Hochbasisgewicht-Bereich in beiden lateralen Seiten des Hochbasisgewicht-Bereichs,
der absorbierende Kern aufweist: einen zentralen biegungsinduzierenden Bereich, der sich in dessen Längsrichtung in Form eines Schlitzes, einer Rille oder eines faserfreien Bereichs in dem Hochbasisgewicht-Bereich erstreckt, und einen seitlichen biegungsinduzierenden Bereich in Form eines faserfreien Bereichs in jedem der Niederbasisgewicht-Bereiche, wobei der faserfreie Bereich ein Bereich ist, wo kein absorbierenden Kern bildendes Material vorhanden ist oder wobei der faserfreie Bereich ein Bereich ist, wo ein absorbierenden Kern bildendes Material in einer Gesamtmenge von 50g/m² oder weniger vorhanden ist,
**dadurch gekennzeichnet, dass**
der zentrale biegungsinduzierende Bereich sich in der Längsrichtung des absorbierenden Kerns hin zu dem vorderen Ende des absorbierenden Kerns über das vordere Ende jedes seitlichen biegungsinduzierenden Bereichs hinaus erstreckt,
die seitlichen biegungsinduzierenden Bereiche sich in der Längsrichtung des absorbierenden Kerns hin zu dem hinteren Ende des absorbierenden Kerns über das hintere Ende des zentralen biegungsinduzierenden Bereichs hinaus erstrecken,
der absorbierende Kern ein absorbierendes Polymer enthält, das absorbierende Element den absorbierenden Kern und eine Kernhüllschicht, die die obere und untere Seite des absorbierenden Kerns bedeckt, aufweist,
in jedem seitlichen biegungsinduzierenden Bereich der über dem absorbierenden Kern liegende Teil der Kernhüllschicht und der unter dem absorbierenden Kern liegende Teil der Kernhüllschicht miteinander verbunden sind, und
in dem zentralen biegungsinduzierenden Bereich der über dem absorbierenden Kern liegende Teil der Kernhüllschicht nicht mit einem genau unter ihm angeordneten Element verbunden ist.

2. Wegwerfwindel nach Anspruch 1, wobei der Hochbasisgewicht-Bereich eine Breite hat, die von einer Zone, wo der zentrale biegungsinduzierende Bereich und die seitlichen biegungsinduzierenden Bereiche in einer nebeneinander liegenden Beziehung sind, hin zu dem vorderen Ende des vorderen Abschnitts zunimmt.

3. Wegwerfwindel nach Anspruch 1 oder 2, wobei das vordere Ende des zentralen biegungsinduzierenden Bereichs nahe an dem vorderen Abschnitt angeordnet ist.

4. Wegwerfwindel nach einem der Ansprüche 1 bis 3, wobei das hintere Ende jedes seitlichen biegungsinduzierenden Bereichs in einer Entfernung L6 von 20 bis 120 cm jenseits des hinteren Endes des zentralen biegungsinduzierenden Bereichs angeordnet ist.

5. Wegwerfwindel nach einem der Ansprüche 1 bis 4, wobei das hintere Ende des zentralen biegungsinduzierenden Bereichs an oder nahe an der Position angeordnet ist, die der Fäzes-Austrittsstelle des Trägers entspricht, und das hintere Ende jedes seitlichen biegungsinduzierenden Bereichs jenseits der Position, die der Fäzes-Austrittsstelle des Trägers entspricht, angeordnet ist.

6. Wegwerfwindel nach einem der Ansprüche 1 bis 5, wobei
der absorbierende Kern eine obere absorbierende Schicht und eine untere absorbierende Schicht, die sowohl in der Längs- als auch Transversalrichtung größer als die obere absorbierende Schicht ist, aufweist,
der Hochbasisgewicht-Bereich ein Bereich ist, wo sich die obere und die untere absorbierende Schicht überlappen,
die Niederbasisgewicht-Bereiche jeweils ein Bereich sind, wo sich die obere und die untere absorbierende Schicht nicht überlappen, und
der zentrale biegungsinduzierende Bereich ein rechteckförmiges Durchgangsloch ist, das länger in der Längsrichtung des absorbierenden Kerns ist und die obere und die untere absorbierende Schicht durchdringt.

7. Wegwerfwindel nach Anspruch 6, wobei das Durchgangsloch das Ergebnis des Stapelns der oberen und unteren absorbierenden Schicht ist, die jeweils ein Durchgangsloch haben und deren Durchgangslöcher miteinander ausgerichtet sind.

8. Wegwerfwindel nach einem der Ansprüche 1 bis 7, wobei der absorbierende Kern in dem Schrittabschnitt einen sich in dessen Längsrichtung erstreckenden zentralen absorbierenden Abschnitt und zwei an beiden lateralen Seiten des zentralen absorbierenden Abschnitts angeordnete seitliche absorbierende Abschnitte hat, und der zentrale absorbierende Abschnitt das absorbierende Polymer in einem höheren Basisgewicht enthält als jeder seitliche absorbierende Abschnitt.

9. Wegwerfwindel nach einem der Ansprüche 1 bis 8, wobei der zentrale absorbierende Abschnitt eine mehrschichtige Struktur hat, die eine obere absorbierende Schicht und eine untere absorbierende Schicht aufweist, und die obere absorbierende Schicht das absorbierende Polymer in einem höheren Basisgewicht enthält als die untere absorbierende Schicht.

10. Wegwerfwindel nach einem der Ansprüche 1 bis 9, die von einem Höschen-Typ ist und eine absorbierende Anordnung mit dem absorbierenden Element und eine Außenhülle aufweist, die sich auf der hautabgewandten Seite der absorbierenden Anordnung befindet und an der die absorbierende Anordnung angebracht ist, wobei sich gegenüberliegende laterale Seitenkanten der Außenhülle in dem vorderen Abschnitt mit denjenigen in dem hinteren Abschnitt verbunden sind, um zwei seitliche Versiegelungen, eine Taillenöffnung und zwei Beinöffnungen zu bilden

11. Wegwerfwindel vom Höschen-Typ nach Anspruch 10, die vom Typ mit einer separierten Außenhülle ist, wobei
die Außenhülle in ein Rückseite-Blatt, das um die Rückseite eines Trägers zu tragen ist, und ein Vorderseite-Blatt, das um die Vorderseite eines Trägers zu tragen ist, separiert ist,
das Hinterseite-Blatt und das Vorderseite-Blatt entlang der zwei Seitenversiegelungen miteinander verbunden sind, um eine zylindrische Form zu bilden, und
die absorbierende Anordnung angebracht ist, um das Vorderseite-Blatt und das Rückseite-Blatt brückenförmig zu verbinden.

12. Wegwerfwindel nach einem der Ansprüche 1 bis 9, die von einem flach ausgebreiteten Typ ist und einen Verschlussstreifen in dem hinteren Abschnitt und einen den Verschlussstreifen empfangenden Landestreifen in der äußeren Seite des vorderen Abschnitts aufweist.

## Revendications

1. Couche jetable comprenant un élément absorbant contenant un cœur absorbant oblong, la couche présentant une portion avant, une portion entre-jambe et une portion arrière adaptées pour être respectivement portées autour de l'avant, de l'entre-jambe et de l'arrière d'un utilisateur,
le cœur absorbant présentant une région à masse surfacique élevée avec une masse surfacique élevée d'un matériau de formation de cœur absorbant dans une partie latéralement intermédiaire de la portion entre-jambe et une région à masse surfacique faible ayant une masse surfacique plus faible du matériau de formation de cœur absorbant que la région à masse surfacique élevée dans les deux côtés latéraux de la région à masse surfacique élevée,
le cœur absorbant présentant une région d'induction de plis centrale s'étendant dans sa direction longitudinale sous la forme d'une fente, d'un sillon ou d'une région dépourvue de fibre dans la région à masse surfacique élevée de celui-ci et une région d'induction de plis latérale sous la forme d'une région dépourvue de fibre dans chacune des régions à masse surfacique faible de celui-ci, dans laquelle la région dépourvue de fibre est une région où aucun matériau de formation de cœur absorbant n'est présent ou dans laquelle la région dépourvue de fibre est une région où un matériau de formation de cœur absorbant est présent en une quantité totale de 50 g/m² ou moins,
**caractérisée en ce que**
la région d'induction de plis centrale s'étendant dans la direction longitudinale du cœur absorbant vers l'extrémité avant du cœur absorbant au-delà de l'extrémité avant de chaque région d'induction de plis latérale,
les régions d'induction de plis latérales s'étendant dans la direction longitudinale du cœur absorbant vers l'extrémité arrière du cœur absorbant au-delà de l'extrémité arrière de la région d'induction de plis centrale,
le cœur absorbant contenant un polymère absorbant, l'élément absorbant comprenant le cœur absorbant et une feuille d'enveloppement de cœur recouvrant le côté supérieur et le côté inférieur du cœur absorbant,
la partie de la feuille d'enveloppement de cœur superposée sur le cœur absorbant et la partie de la feuille d'enveloppement de cœur sous-jacent au cœur absorbant étant liées l'une à l'autre dans chaque région d'induction de plis latérale, et
la partie de la feuille d'enveloppement de cœur superposée sur le cœur absorbant n'est pas liée à un élément situé juste dessous dans la région d'induction de plis centrale.

2. Couche jetable selon la revendication 1, dans laquelle la région à masse surfacique élevée présente une largeur augmentant d'une zone où la région d'induction de plis centrale et les régions d'induction de plis latérales sont dans une relation juxtaposée vers l'extrémité avant de la portion avant.

3. Couche jetable selon la revendication 1 ou 2, dans laquelle l'extrémité avant de la région d'induction de plis centrale est en-deçà de la portion avant.

4. Couche jetable selon l'une quelconque des revendications 1 à 3, dans laquelle l'extrémité arrière de chaque région d'induction de plis latérale est au-delà de l'extrémité arrière de la région d'induction de plis centrale d'une distance L6 de 20 à 120 cm.

5. Couche jetable selon l'une quelconque des revendications 1 à 4, dans laquelle l'extrémité arrière de la région d'induction de plis centrale est au niveau ou en-deçà de la position correspondant au point de sortie des excréments de l'utilisateur, et l'extrémité arrière de chaque région d'induction de plis latérale est au-delà de la position correspondant au point de sortie des excréments de l'utilisateur.

6. Couche jetable selon l'une quelconque des revendications 1 à 5, dans laquelle le cœur absorbant comprend une couche absorbante supérieure et une couche absorbante inférieure plus grande que la couche absorbante supérieure dans la direction longitudinale et la direction transversale, la région à masse surfacique élevée est une région où les couches absorbantes supérieure et inférieure se chevauchent, les régions à masse surfacique faible sont chacune une région où les couches absorbantes supérieure et inférieure ne se chevauchent pas, et la région d'induction de plis centrale est un trou traversant rectangulaire plus long dans la direction longitudinale du cœur absorbant et perçant à travers les couches absorbantes supérieure et inférieure.

7. Couche jetable selon la revendication 6, dans laquelle le trou traversant est le résultat de l'empilement des couches absorbantes supérieure et inférieure présentant chacune un trou traversant avec leurs trous traversants alignés l'un sur l'autre.

8. Couche jetable selon l'une quelconque des revendications 1 à 7, dans laquelle le cœur absorbant présente, dans la portion entre-jambe, une portion absorbante centrale s'étendant dans sa direction longitudinale et une paire de portions absorbantes latérales situées sur les deux côtés latéraux de la portion absorbante centrale, et la portion absorbante centrale contient le polymère absorbant dans une masse surfacique plus élevée que chaque portion absorbante latérale.

9. Couche jetable selon l'une quelconque des revendications 1 à 8, dans laquelle la portion absorbante centrale comporte une structure multicouche comprenant une couche absorbante supérieure et une couche absorbante inférieure, et la couche absorbante supérieure contient le polymère absorbant dans une masse surfacique plus élevée que la couche absorbante inférieure.

10. Couche jetable selon l'une quelconque des revendications 1 à 9, qui est du type à enfiler, comprenant un ensemble absorbant comportant l'élément absorbant et un revêtement extérieur situé sur le côté non tourné vers la peau de l'ensemble absorbant et présentant l'ensemble absorbant fixé à celui-ci, le revêtement extérieur ayant ses côtés latéraux opposés dans la portion avant reliés à ceux dans la portion arrière pour former une paire de joints latéraux, une ouverture de taille et une paire d'ouvertures pour les jambes.

11. Couche à enfiler jetable selon la revendication 10, qui est du type à revêtement extérieur séparé dans laquelle le revêtement extérieur est séparé en un panneau latéral arrière adapté pour être porté autour du côté arrière d'un utilisateur et un panneau latéral avant adapté pour être porté autour du côté avant d'un utilisateur, le panneau latéral arrière et le panneau latéral avant étant reliés l'un à l'autre le long de la paire de joints latéraux pour former une forme cylindrique, et l'ensemble absorbant étant fixé pour enjamber le panneau latéral avant et le panneau latéral arrière.

12. Couche jetable selon l'une quelconque des revendications 1 à 9, qui est de type plat présentant une bande de fixation dans la portion arrière et une bande d'appui recevant la bande de fixation dans le côté extérieur de la portion avant.
